# EUROPEAN PATENT APPLICATION

(11) **EP 4 666 976 A1**
(43) Date of publication of application: **24.12.2025**
(21) Application number: 25183339.8
(22) Date of filing: 17.06.2025
(51) Int. Cl.: A61B 34/10, A61B 34/20, A61B 34/30

(54) **DYNAMIC DEVICE MANIPULATION**

(30) Priority: 21.06.2024 US 202418749886
(71) Applicant: Siemens Healthineers Endovascular Robotics, Inc., Newton, MA 02466 (US)
(72) Inventor: CANALE, Cameron, Groton, MA, 01450 (US); SOKHANVAR, Saeed, Belmont, MA, 02478 (US); CLARK, Andrew, Arlington, MA, 02476 (US)
(74) Representative: HKW Intellectual Property PartG mbB

(57) **Abstract**

A controller includes at least one processor and memory coupled to the at least one processor. The memory stores computer-executable instructions. The at least one processor is configured to execute the computer-executable instructions to cause the controller to determine a clinical driving scenario associated with a percutaneous device, and set driving characteristics associated with the percutaneous device based on the clinical driving scenario and information included in a driving profile, wherein the information included in the driving profile includes information associating particular clinical driving scenarios with particular driving characteristics.

## Description

### TECHNICAL FIELD

This application relates generally to manipulating movements of percutaneous devices, and more specifically to dynamically manipulating movement of percutaneous devices.

### BACKGROUND

Percutaneous devices, for example catheters, are often used in conjunction with bedside systems that allow a physician to control movement of the percutaneous devices using a driving interface.

### SUMMARY

The scope of protection sought for various example embodiments is set out by the independent claims. The example embodiments and/or features, if any, described in this specification that do not fall under the scope of the independent claims are to be interpreted as examples useful for understanding various embodiments.

Independent of the grammatical term usage, individuals with male, female or other gender identities are included within the term.

In various example embodiments, a controller includes at least one processor and memory coupled to the at least one processor. The memory stores computer-executable instructions. The at least one processor is configured to execute the computer-executable instructions to cause the controller to determine a clinical driving scenario associated with a percutaneous device, and set driving characteristics associated with the percutaneous device based on the clinical driving scenario and information included in a driving profile. The information included in the driving profile includes information associating particular clinical driving scenarios with particular driving characteristics. In some example embodiments, the at least one processor is further configured to execute the computer-executable instructions to cause the controller to determine the clinical driving scenario based on positional information indicating a distance the percutaneous device is inserted in a patient.

The at least one processor may be further configured to execute the computer-executable instructions to cause the controller to determine the clinical driving scenario based on a comparison of the distance the percutaneous device is inserted into the patient and an estimated patient vascular length. In some such example embodiments, the positional information includes at least one of, information indicating a position of an arm of an imaging system relative to a patient, a location of the controller relative to the patient, a position of a cartridge along a drive-axis of the controller, image recognition of a marker on the patient, or image recognition of a marker on the percutaneous device.

In any or all of the above example embodiments, the at least one processor is further configured to execute the computer-executable instructions to cause the controller to receive user control signals from a driver interface, wherein the user control signals indicate requested movement of a percutaneous device being controlled by the controller, and set the driving characteristics of the percutaneous device by modifying the user control signals based on the clinical driving scenario and the information included in the driving profile. The at least one processor may be further configured to execute the computer-executable instructions to cause the controller to determine the clinical driving scenario based on at least one of a field of view of an imaging system or a position of an imaging system relative to a patient, wherein the field of view of the imaging system represents a zone between an access site at which the percutaneous device is inserted into the patient and the head of the patient.

In some example embodiments, the information included in the driving profile includes information defining, establishing, imposing, and/or limiting the particular driving characteristics under the particular clinical driving scenarios. The particular clinical driving scenarios may include at least one of a type of percutaneous device, a configuration of the percutaneous device, a distance the percutaneous device is inserted in a patient, or whether a distal portion of the percutaneous device is still fully within an existing percutaneous device. The particular driving characteristics may include at least one of a peak velocity, a peak acceleration, jerkiness, a peak rotational velocity, a peak rotational acceleration, a displacement per input ratio, a maximum absolute displacement, a maximum force, a maximum linear displacement, or a rotational lockout. As used herein, the term, "jerk" and its derivative terms refer to the time rate change of acceleration.

In various example embodiments, including any or all of the above example embodiments, a controller includes at least one processor and memory coupled to the at least one processor. The memory stores computer-executable instructions, and the at least one processor is configured to execute the computer-executable instructions to cause the controller to determine a distance a percutaneous device is inserted in a patient, receive user control signals from a driver interface, wherein the driver interface is configured to respond to user interaction with the driver interface to control movement of the percutaneous device, generate adjusted user control signals based on the distance the percutaneous device is inserted in the patient, and transmit the adjusted user control signals to a motive device configured to move the percutaneous device in accordance with the adjusted user control signals.

In some such example embodiments, the at least one processor is further configured to execute the computer-executable instructions to cause the controller to determine the distance the percutaneous device is inserted in the patient based at least in part on kinematic information, on a position of an imaging system relative to a patient, and/or on a location of a percutaneous device manipulation system relative to the patient. In some example embodiments, the at least one processor is further configured to execute the computer-executable instructions to cause the controller to determine the distance the percutaneous device is inserted in the patient based, at least in part, on a displacement of a cartridge along a drive-axis of a percutaneous device manipulation system.

In any or all of the above example embodiments, the at least one processor is further configured to execute the computer-executable instructions to cause the controller to generate the adjusted user control signals by altering the user control signals based on at least one of a field of view of an imaging system, or a position of an imaging system relative to a patient, wherein the field of view of the imaging system represents a zone between an access site at which the percutaneous device is inserted into the patient and the head of the patient.

In some such example embodiments, the at least one processor is further configured to execute the computer-executable instructions to cause the controller to generate the adjusted user control signals based on profile information included in a driving profile, wherein the profile information included in the driving profile includes first information associating the distance the percutaneous device is inserted in the patient with movement characteristics of the percutaneous device.

In some such example embodiments, the profile information included in the driving profile further includes second information associating the movement characteristics of the percutaneous device with at least one of a percutaneous device safe-loading position, a percutaneous device type, or a configuration of the percutaneous device. In various example embodiments, the movement characteristics of the percutaneous device include at least one of a peak velocity limit, a peak acceleration limit, a jerkiness limit, a rotational velocity limit, a displacement per input ratio, an absolute displacement limit, a rotational lockout, a maximum force limit, or a linear displacement limit.

In various example embodiments, including various combinations of the above example embodiments, a robotic device includes a motor configured to impart motion to a percutaneous device in response to motor control signals, a driver interface configured to transmit user control signals to a controller in response to user manipulation of the driver interface, and a controller coupled to the motor and the driver interface. In some such example embodiments, the controller is configured to determine a clinical driving scenario associated with the robotic device, wherein the clinical driving scenario includes a distance the percutaneous device is inserted in a patient, set driving characteristics of the robotic device based on the clinical driving scenario and information included in a driving profile, wherein the information included in the driving profile includes information associating particular clinical driving scenarios with particular driving characteristics, and generate the motor control signals based on the driving characteristics.

In some such example embodiments, the motor is further configured to impart the motion to the percutaneous device by moving a cartridge coupled to the motor along a drive axis in response to the motor control signals, wherein movement of the cartridge moves the percutaneous device.

In some example embodiments, a controller includes means for determining a distance a percutaneous device is inserted in a patient, receiving user control signals from a driver interface, wherein the driver interface is configured to respond to user interaction with the driver interface to control movement of the percutaneous device, generating adjusted user control signals based on the distance the percutaneous device is inserted in the patient, and transmitting the adjusted user control signals to a motive device configured to move the percutaneous device in accordance with the adjusted user control signals.

Any or all of the above example embodiments, and other example embodiments disclosed herein, may be used in various combinations to dynamically manipulate movement of a percutaneous device.

### BRIEF DESCRIPTION OF THE DRAWINGS

Example embodiments will become more fully understood from the detailed description given herein below and the accompanying drawings, wherein like elements are represented by like reference numerals. The example embodiments are given by way of illustration only, and thus are not limiting of this disclosure.
FIG. 1 is a diagram of a system, in accordance with various example embodiments;
FIG. 2 is a diagram illustrating a percutaneous device inserted into a patent, in accordance with various example embodiments;
FIG. 3 is a diagram of a percutaneous device manipulation system, in accordance with various example embodiments;
FIG. 4 is a diagram of percutaneous device stacks, in accordance with various example embodiments;
FIG. 5 is a flowchart illustrating a method, in accordance with various example embodiments;
FIG. 6 is a flowchart illustrating a method, in accordance with various example embodiments;
FIG. 7 is a flowchart illustrating a method, in accordance with various example embodiments;
FIG. 8 is a flowchart illustrating a method, in accordance with various example embodiments;
FIG. 9 is a flowchart illustrating a method, in accordance with various example embodiments;
FIG. 10 is a flowchart illustrating a method, in accordance with various example embodiments;
FIG. 11 is a flowchart illustrating a method, in accordance with various example embodiments;
FIG. 12 is a flowchart illustrating a method, in accordance with various example embodiments;
FIG. 13 is a diagram illustrating an imaging system field of view (FoV) location in relation to table position, in accordance with various example embodiments;
FIG. 14 is a diagram illustrating an imaging system field of view (FoV) location in relation to a percutaneous device manipulation system, in accordance with various example embodiments;
FIG. 15 is a diagram illustrating an imaging system field of view (FoV) location in relation patient height and/or table position, in accordance with various example embodiments;
FIG. 16 is a series of graphs illustrating percutaneous device driving characteristics, in accordance with various example embodiments;
FIGS. 17A and 17B illustrate adjusting a control-resolution of percutaneous device movements using detents, in accordance with various example embodiments;
FIG. 18 is a graph illustrating positional control clipping, in accordance with various example embodiments;
FIG. 19 is a graph illustrating clipped velocity control signals, in accordance with various example embodiments;
FIG. 20 is a block diagram of a percutaneous device control system, in accordance with various example embodiments; and
FIG. 21 is a block diagram of a processing system, in accordance with various example embodiments.

It should be noted that these figures are intended to illustrate the general characteristics of methods, structure and/or materials utilized in certain example embodiments and to supplement the written description provided below. These drawings are not, however, to scale and may not precisely reflect the precise structural or performance characteristics of any given embodiment, and should not be interpreted as defining or limiting the range of values or properties encompassed by example embodiments. The use of similar or identical reference numbers in the various drawings is intended to indicate the presence of a similar or identical element or feature.

### DETAILED DESCRIPTION

Various example embodiments will now be described more fully with reference to the accompanying drawings in which some example embodiments are shown.

Detailed illustrative embodiments are disclosed herein. However, specific structural and functional details disclosed herein are merely representative for purposes of describing example embodiments. The example embodiments may, however, be embodied in many alternate forms and should not be construed as limited to only the embodiments set forth herein.

It should be understood that there is no intent to limit example embodiments to the particular forms disclosed. On the contrary, example embodiments are to cover all modifications, equivalents, and alternatives falling within the scope of this disclosure. Like numbers refer to like elements throughout the description of the figures. One or more example embodiments described herein may be combined.

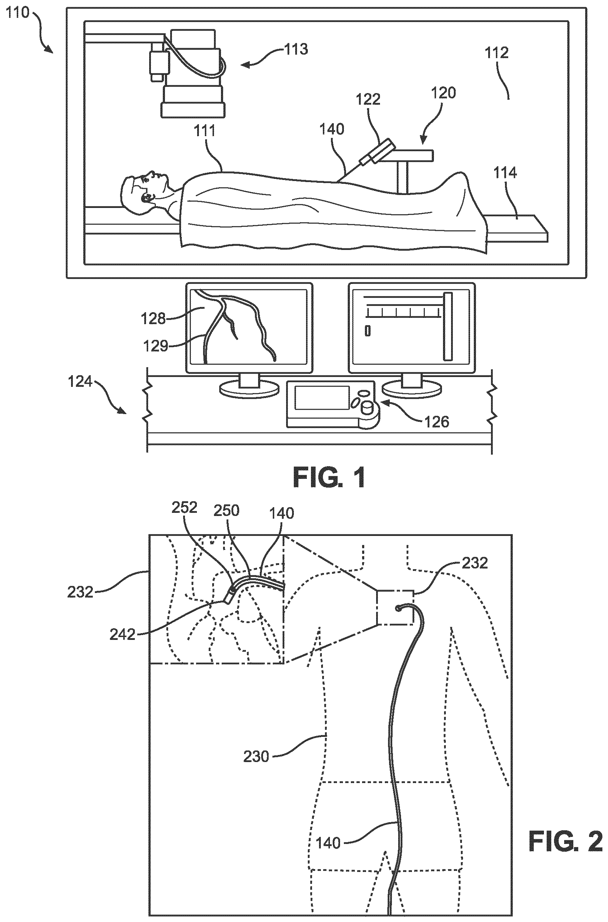

Referring first to FIG. 1 a system 110 will be discussed in accordance with various example embodiments. In the illustrated example embodiments, system 110 includes a bedside system 112 for robotically performing percutaneous interventional procedures under control of a medical professional.

As illustrated in FIG. 1, a patient 111 is supported on a table 114. The medical professional may observe the procedure using imaging equipment 113, for example fluoroscopic X-ray equipment, included in bedside system 112. A cassette 122 supported by a robotic arm 120 may be used to automatically feed a guide wire 250 (shown in FIG. 2) into a guide catheter 140 within the body of the patient 111. The cassette 122 may be controlled from a remote station 124 in order to isolate the medical personnel conducting the procedure from exposure to the X-ray radiation used to monitor the procedure by use of fluoroscopic equipment. In various example embodiments, the remote station 124 includes remote controls 126 for controlling the cassette 122 and a screen 128 with which to monitor the progress of the procedure. In the illustrated example embodiment, screen 128 displays the arterial system 129 being addressed by the procedure.

Referring next to FIG. 2, placement of a percutaneous device within patient 111 will be discussed in accordance with various example embodiments. As illustrated, a guide catheter 140 that has been fed into the torso 230 of a patient 111 to reach the cardiac region 232. Within the guide catheter 140 is a guide wire 250 whose tip 252 has not yet passed out of a distal end 242 of the guide catheter 140. The imaging equipment 113 may be used to monitor the progress of the guide wire 250 as it passes through the guide catheter 140 and approaches its distal terminus 242. In some example embodiments as one or more device tips approach a region of interest 252, the field of view of the imaging equipment 113 may be zoomed. The imaging equipment 113 may also be instructed to capture images at a more frequent rate once the tip 252 enters the region of interest 232.

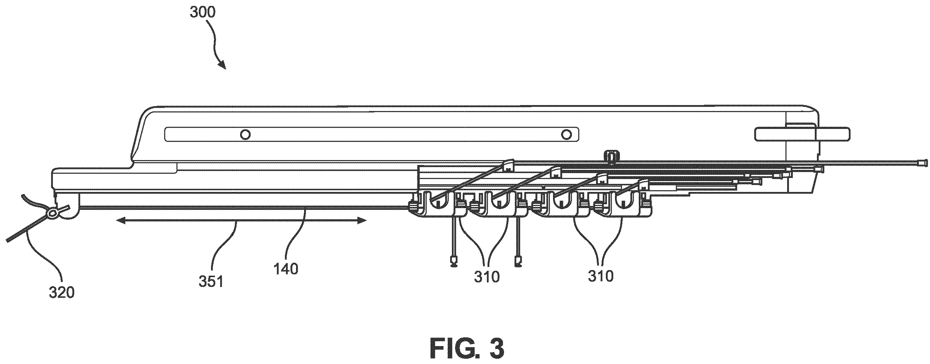

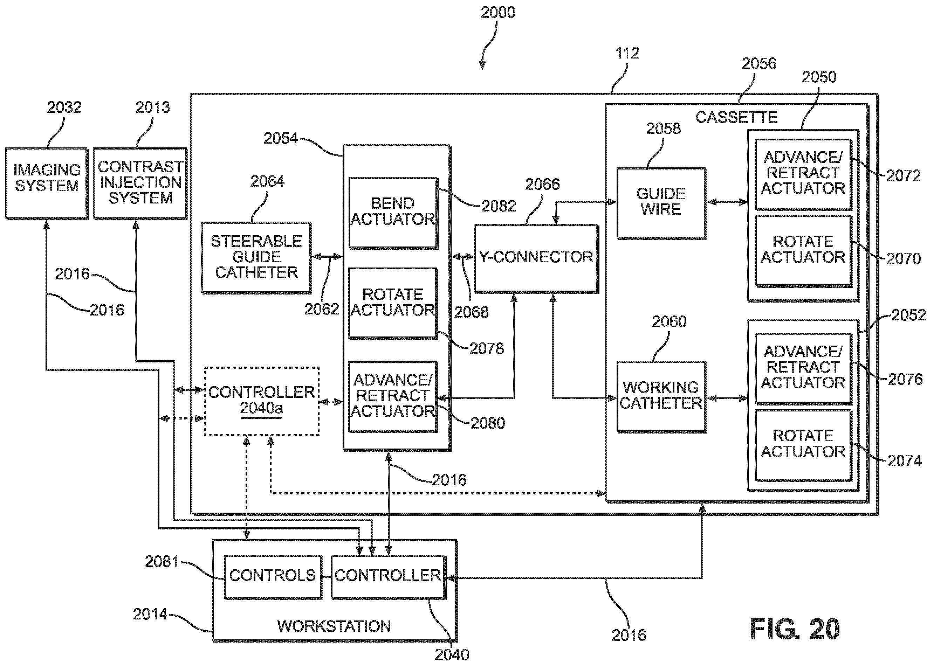

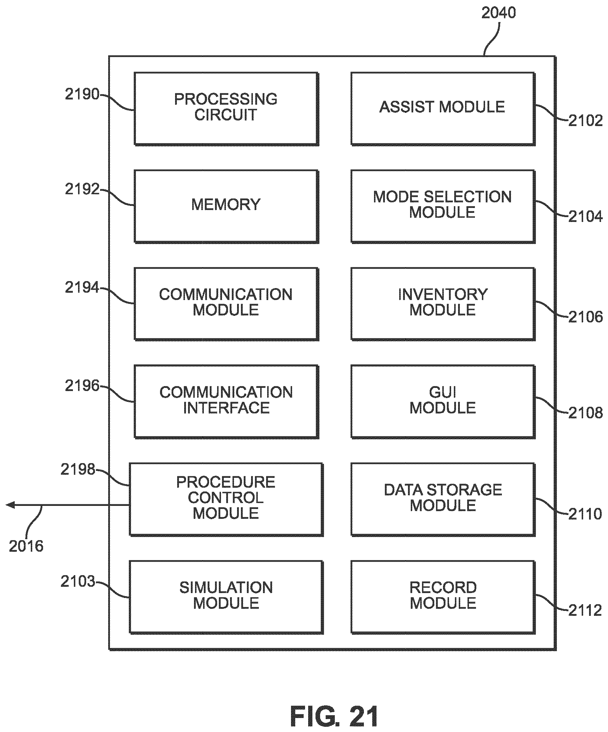

FIG. 3 is a diagram of a percutaneous device manipulation system 300 that will be discussed in accordance with various example embodiments. Percutaneous device manipulation system 300 includes a number of cartridges/cassettes 310, connected to guide catheter 140 having an introducer sheath at its distal end. A cartridge/cassette 310 connected to guide catheter 140 may be used to impart movement to guide catheter 140 as the cartridge/cassette 310 moves along and/or rotates in relation to drive axis 351. Other cartridges/cassettes 310 may be connected to working catheters, child guiding catheters, or the like, placed within guide catheter 140 (not illustrated), such that each cartridge/cassette 310 may impart motion, linear and/or rotational, to different catheters/subcutaneous devices. Additional discussion regarding the operation of percutaneous device manipulation system 300 is provided subsequently in the discussion of FIGS. 20 and 21.

In at least one example embodiment, kinematic information regarding the position of cartridges/cassettes 310 in relationship to their starting positions can be obtained from sensors that are included in percutaneous device manipulation system 300, and in many cases part of each individual cartridge/cassette 310. For example, linear encoders may be used to determine a linear distance traveled by a cartridge/cassette 310. Linear encoders can include, for example, optical and/or mechanical readers that determine the linear distance travelled by monitoring a number of markers, slots, on a scale over which the sensor passes, or by otherwise detecting a position of the cartridge/cassette 310 relative to the scale.. Linear and/or rotational positions may be determined using worm gears, sprockets, or the like may be used in some example embodiments of cartridges/cassettes 310. In some such example embodiments, rotational sensors can be used to monitor the number of rotations of gears or sprockets, and that information may be used to determine an amount of displacement along a drive axis 351. The rate of rotation and/or the change in the rate of rotation of a percutaneous device can also be measured or computed to determine velocity, acceleration, angular velocity, angular acceleration, direction of movement, and the like. Other sensors, such as optical sensors that employ through beam, specular, or diffuse reflective techniques, and other mechanical or electronic sensors may be used in various example embodiments. In various example embodiments, the kinematic information is transmitted to a controller, which may use the information to determine a distance the percutaneous device is inserted in a patient.

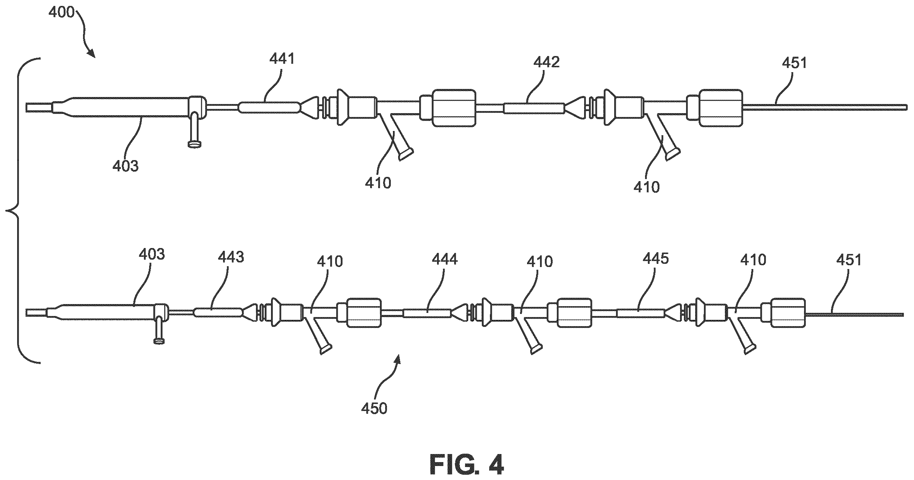

Referring next to FIG. 4 percutaneous device stacks 400 and 450 will be briefly discussed in accordance with various example embodiments. In various example embodiments, multiple percutaneous devices may be combined, or stacked, depending on the procedure being performed. The types of devices used for particular procedures is beyond the scope of this disclosure. However, in some example embodiments the number of stacked devices, the type of stacked devices, and the type of procedure being performed, impact desired driving characteristics of the percutaneous device are factors used in determining a clinical driving scenario associated with particular percutaneous device driving characteristics.

In the illustrated example embodiment, a first device stack 400 includes two stacked percutaneous devices, guide catheter 441 and working catheter 442. Each of the guide catheter 441 and working catheter 442 are connected to separate Y -connectors 410. An insertion guide 403 and a guide wired 451 are also included in the first device stack 400. One end of guide catheter 441 is connected to a Y connector 410, one end of working catheter 442 is connected to the second Y connector 410, and the other end is positioned inside of guide catheter 441. Guide wire 451 is positioned inside of working catheter 442, and is therefore also within guide catheter 441. In various example embodiments, the first device stack may be locked into one or more cartridges/cassettes 310.

In the illustrated example embodiment, a second device stack 450 includes three stacked percutaneous devices, guide catheter 443 and mother catheter 444, and child catheter 445. As with first device stack 450, each of the catheters is connected to a Y-connectors 410, and each catheter is "nested" with the adjacent catheter. The percutaneous devices included in second device stack 450 may be different from the percutaneous devices included in first device stack 400.

In at least some example embodiments, a medical professional may input the type and number of percutaneous devices included in the device stack selected for use, and that information may be used as input to a control configured to determine a clinical driving scenario associated with percutaneous device driving characteristics. In some example embodiments, the information may be entered via direct user input, using a scanning device to scan a barcode or QR code, using device SKUs to reference an internal database for parameters like device length, inner diameter/outer diameter (ID/OD), device class/category, etc.

In at least one example embodiment, a single clinical driving scenario may be associated with the entire stack, but the driving characteristics associated with individual percutaneous devices included in the stack may be different. Some considerations regarding selection of clinical driving scenarios for stacks can include, but are not limited to, the information included in Table 1.

**Table 1**

| Stack | Manipulation Consideration |
|---|---|
| Diagnostic : GW | Devices do not go distal into neuro anatomy. Able to manipulate at higher speeds |
| Base: Diagnostic: GW | This combination of devices does not go distal into neuro anatomy. |
| | Able to manipulate at higher speeds |
| Base: Aspiration : MicroCath: Micro GW | Because of the microcatheter and micro guide wire, this stack should be manipulated at lower speeds |

In some example embodiments, when a device or device stack is in a safe loading position, for example when percutaneous device is fully within another device, fully protected, and not exposed in the anatomy, the percutaneous device may be permitted to travel at higher speeds.

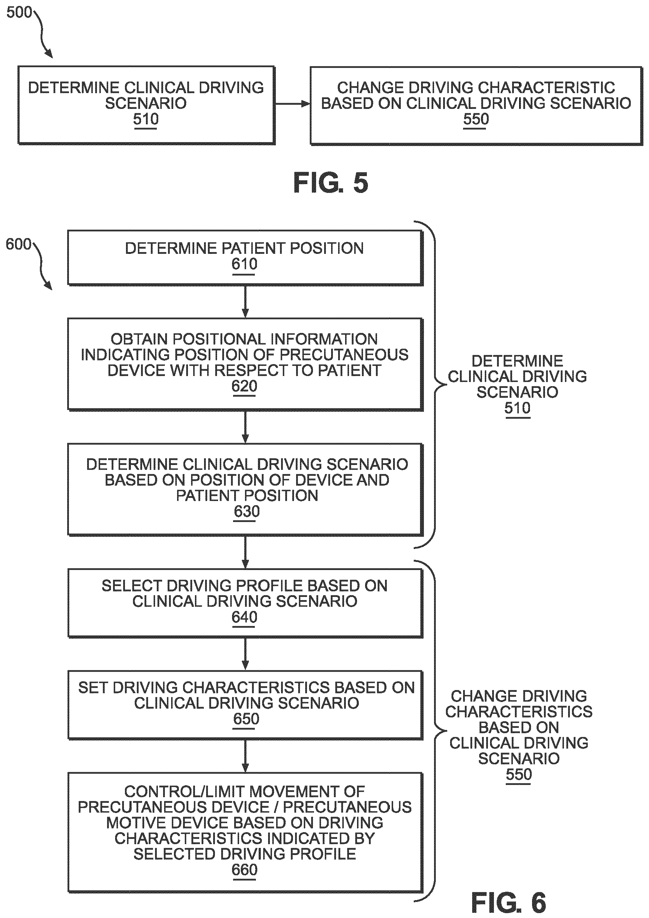

Methods in accordance with various example embodiments will be discussed with reference to FIGS. 5-12. Any or all of the methods discussed with reference to FIGS. 5-12 may be performed by a controller, such as controller 1940 subsequently illustrated and discussed with respect to FIG. 21, or some other suitable processing device and/or system.

Referring next to FIG. 5 a method 500 will be discussed in accordance with various example embodiments. As illustrated by method 500, a clinical driving scenario may be determined at block 510. In various example embodiments, the clinical driving scenario may be determined based, at least in part on kinematic information obtained from the a percutaneous device manipulation system, kinematic information obtained from a moveable table, patient position, a size of a patient, estimated or measured vascular length of the patient, the number and types of devices in a stack, a field of view (FoV) of an imaging system, a position of an imaging system (or some portion of the imaging system), a distance the percutaneous device has travelled into the patient, whether a device stack is in a safe loading position, a part of the body in which the percutaneous device is located, or is about to be located, some combination of the above information.

In at least some example embodiments, one or more driving profiles may be suggested, and the medical personnel performing the procedure may select a driving scenario from among the offered choices. In some example embodiments, driving profiles may be user customizable. In various example embodiments, a controller may automatically adjust the driving scenario without manual input from the operator, with a potential emergency manual override being available in some example embodiments.

As illustrated by block 550, the driving characteristics of a percutaneous device may be set or altered based on the clinical driving scenario. In various example embodiments, one or more driving profiles are stored in a memory accessible to a controller of a percutaneous device manipulation system 300 (FIG. 3). The controller and/or the memory may be part of percutaneous device manipulation system 300, part of a computing device, such as remote station 124 (FIG. 1) used to accept driver input from a medical professional operating percutaneous device manipulation system 300, or in another suitable location. A controller according to various example embodiments is discussed further with reference to FIGS. 20 and 21.

In at least one example embodiment, a driving profile may be stored in one or more memories, for example memory 2192 (FIG. 21). Some portions of the driving profile may be stored on are remote computing device accessible via a communications network, for example in a cloud-based storage server, in a memory included in the bedside system 112, in remote station 124, or otherwise. A driving profile may be stored in a data structure known to those of ordinary skill in the art, including, but not limited to, a relational database, in a lookup table, a tree, an array, a heap, or the like. In various example embodiments, the driving profile includes information linking one or more driving scenarios to particular driving characteristics.

For example, a first driving scenario may specify that a percutaneous device of a first type inserted 50 cm into a 6-foot-tall patient may have a first maximum allowable linear velocity, is prohibited from rotating, have a first maximum acceleration, and have a control signal multiplier of 1. In some example embodiments, the control signal multiplier may be a factor by which an input signal from a driver is multiplied to generate a corrected signal.

Continuing with the same example, a second driving scenario may specify that the same percutaneous device inserted 150 cm into the same 6-foot-tall patient has a reduced maximum allowable linear velocity, is allowed to rotate up to 30 degrees in either direction, have reduced maximum acceleration, and have a control signal multiplier of .6. By reducing the control signal multiplier, and input signal of, for example 1 mV generated by the driver input device will be "smoothly" reduced to .6 mV. In other example embodiments, as discussed later herein, rather than a multiplier, the original control signals may be clipped. As used herein, clipping a signal includes, but is not limited to, restricting a maximum instantaneous and/or average voltage or current of a signal to a maximum level. Clipping can include, for example, limiting a peak and/or a root-mean-square voltage of a sinusoidal or other varying waveform by using a diode or other clipping circuit to shunt a portion of the signal to ground. Various techniques for clipping digitized waveforms and/or non-varying are also known in the art. For example, a duty cycle of a digital waveform may be limited, effectively clipping the average voltage of a control signal.

In some such example embodiments, a driving profile links the selected driving scenarios to driving characteristics, and automatically sets or adjusts, the driving characteristics associated with the percutaneous device until a new driving scenario is selected.

Referring next to FIG. 6 a method 600 will be discussed in accordance with various example embodiments. In the illustrated example embodiment, blocks 610, 620, and 630 are sub tasks of block 510, while blocks 640, 650, and 660 are sub tasks of block 550. As illustrated by block 610, a position of a patient may be determined. In various example embodiments, the position of the patent can be determined based on kinematic information from a moveable table, based on an imaging system, based on size/height of the patient, or the like.

As illustrated by block 620, positional information indicating a position of the subcutaneous device with respect to patient is obtained according to some example embodiments. This information may be obtained based on a position of a percutaneous device manipulation system 300 relative to position of the patient in combination is the distance the cartridge has moved along a drive axis of the percutaneous device manipulation system 300, based on information obtained from an imaging system, or the like.

As illustrated by block 630, a clinical driving scenario is determined based on the results of block 620. For instance, in some example embodiments, there may be three driving scenarios. It will be appreciated that there may be substantially more than three potential driving scenarios, but three is chosen for ease of illustration. A first driving scenario may be used when the percutaneous device is between about 0 cm and 40 cm inside of the patient, a second driving scenario may be used when the percutaneous device is between about 40 cm and 70 cm inside the patient, and a third driving scenario may be used when the percutaneous device is between about 70 cm and 90 cm inside of the patient. In such a case, if the percutaneous device is 10 cm inside the patient, the second driving scenario will be used. It will be appreciated that in at least some example embodiments, catheters of approximately 165 cm may be used, but the entire length of the catheter may not be inserted into the patient. The above discussion is used to provide an example, and is not intended to limit the different driving scenarios to only the stated ranges.

As illustrated by block 640, a driving profile is selected based on the clinical driving scenario. Continuing with the previous example, multiple driving profiles may be available, with each driving profile being associated with one or more driving scenarios. Note that in some embodiments there is not a one-to-one correspondence between driving scenarios and driving profiles. For example, a single driving profile might be associated with multiple driving scenarios but each driving scenario may be associated with only a single driving profile. For example, there may be two driving scenarios that permit the same maximum speed and acceleration, thus two driving scenarios may be associated with a single driving profile. However, in at least one example embodiment, a single driving profile will not permit two different maximum speeds (at least for the same device).

As illustrated by block 650, the controller may set the driving characteristics specified in the driving profile. Because the driving profile is selected based on the driving scenario, it can be said that the driving characteristics are based on the driving scenario - the driving scenario ultimately determines the driving characteristics in some example embodiments.

As illustrated by block 660, movement of percutaneous device/percutaneous motive device may be controlled/limited based on driving characteristics indicated by selected driving profile.

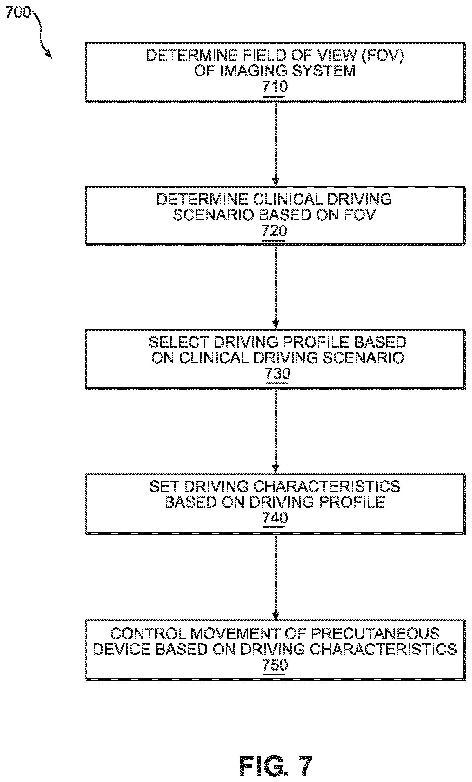

Referring next to FIG. 7 a method 700 will be discussed in accordance with various example embodiments. As illustrated by block 710, a Field of View (FOV) of an imaging system, *e.g.,* imaging equipment 113 (FIG. 1), is determined. Information indicating the FoV of an imaging system may be provided to a controller, which can use that information to make the determination. For example, the imaging system may directly communicate coordinates of its FoV to the controller, the controller may determine the FoV of the imaging system based on a location of the imaging system in relation to a patient, and indirectly from the imaging system's location relative to a table on which the patient is positioned. In some example embodiments, a controller responsible for setting the imaging system's FoV may provide the information to the controller responsible for determining the clinical drive scenario. In some example embodiments, the imaging system controller and the controller responsible for determining the clinical drive scenario are one and the same.

As illustrated by block 720, a clinical driving scenario is determined based on the FoV of the imaging system. For example, if the FoV of the imaging system is greater than about 70 cm, a first driving scenario corresponding to a drive profile that allows faster lateral movement may be selected. A second driving scenario, corresponding to a drive profile that limits lateral movement speed, may be chosen if the FoV of the imaging system is less than 10 cm.

As illustrated by block 730, a driving profile is selected based on the clinical driving scenario. In various example embodiments, a table or database may be used to link clinical driving scenarios to driving profiles. In various example embodiments, as illustrated by block 740, driving characteristics are set based on the driving profile, and movement of the percutaneous device is controlled base on the driving characteristics, as illustrated by block 750.

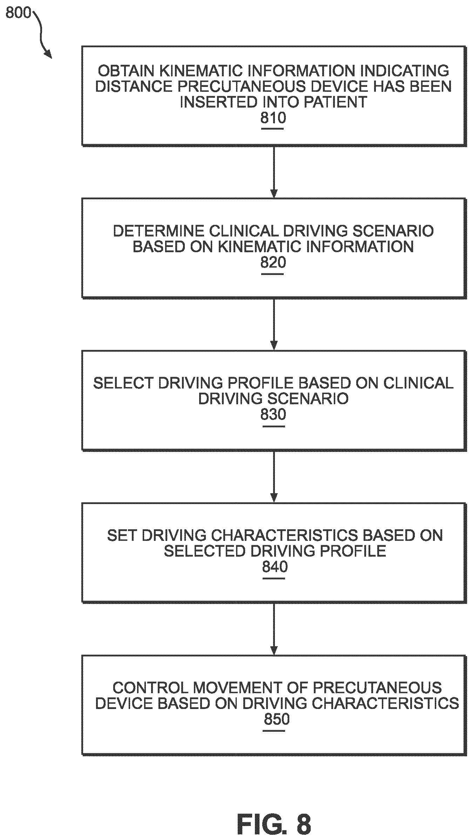

Referring next to FIG. 8, a method 800 will be discussed in accordance with various example embodiments. As illustrated by block 810, kinematic information indicating distance subcutaneous device has been inserted into patient is obtained. Kinematic information includes, but is not limited to, information regarding a position of a movable table, information indicating a position of a cartridge along a movement axis, positional information associated with an imaging system, or the like. As illustrated by block 820, a clinical driving scenario is determined based on the kinematic information, and a driving profile is selected based on the clinical driving scenario, as illustrated by block 830. As illustrated by block 840, driving characteristics are selected base on the driving profile, and as illustrated by block 850, movement of the percutaneous device is controlled base on the driving characteristics.

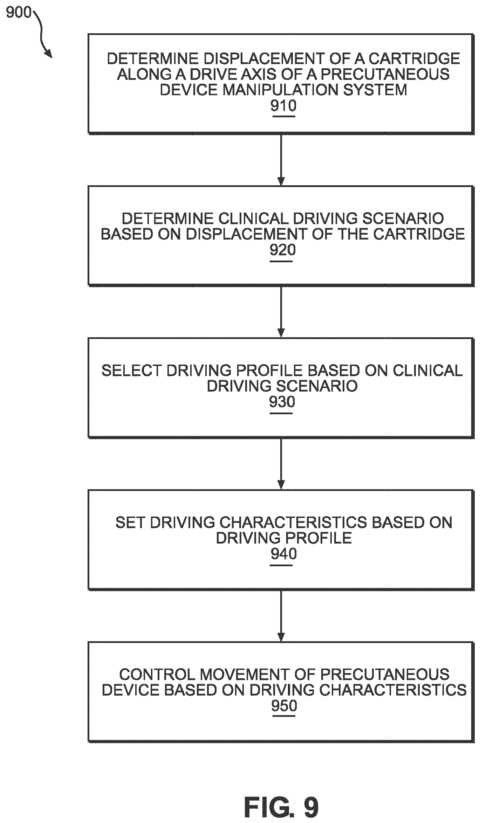

Referring next to FIG. 9, a method 900 will be discussed in accordance with various example embodiments. As illustrated by block 910, a displacement of a cartridge along a drive axis of a percutaneous device manipulation system is determined. As illustrated by block 920, a clinical driving scenario is determined based on the displacement of the cartridge. As illustrated by block 930, a driving profile is selected based on the clinical driving scenario. At block 940 driving characteristics are set based on the driving profile, and as illustrated by block 950, movement of the percutaneous device is controlled based on the driving characteristics.

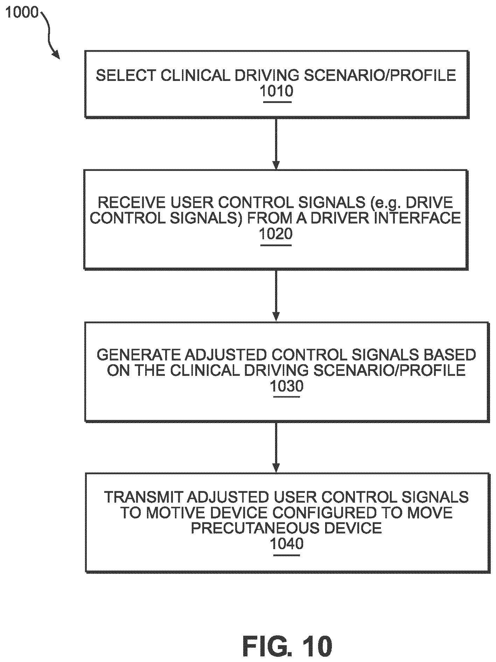

Referring next to FIG. 10, a method 1000 will be discussed in accordance with various example embodiments. As illustrated by block 1010, a clinical scenario/driving profile is selected. Selection of the clinical driving profile may be performed using any of various techniques, or combinations of techniques, disclosed in conjunction with the above example embodiments. In some example embodiments, selection of the clinical driving scenario and selection of the driving profile constitute a single-step action, such that selection of the clinical driving scenario is coextensive with selecting the driving profile.

As illustrated by block 1020, a controller receives movement control signals, for example drive control signals, from a medical professional interacting with a driver interface. As illustrated by block 1030, the controller adjusts the receive control signals and generates adjusted control signals based on the clinical scenario/driving profile and their associated driving characteristics. As discussed elsewhere herein, in various example embodiments, the adjusted control signals may be clipped, have a negative or positive gain applied, filtered, blocked, or otherwise modified either digitally or in an analog manner. For example, if a maximum speed has been reached, a user requested "speed increase" signal may be immediately clipped. In another example embodiment, if a percutaneous device is accelerating too quickly, but has not reached a maximum speed, a user requested "speed increase" signal may be "muted," or have a negative gain applied to allow acceleration, but at a reduced rate.

As illustrated by block 1040, the adjusted/modified control signal may be transmitted to a motive device in place of the original control signal. Additional discussion of control signals and various motive devices is provided with reference to FIGS. 20 and 21.

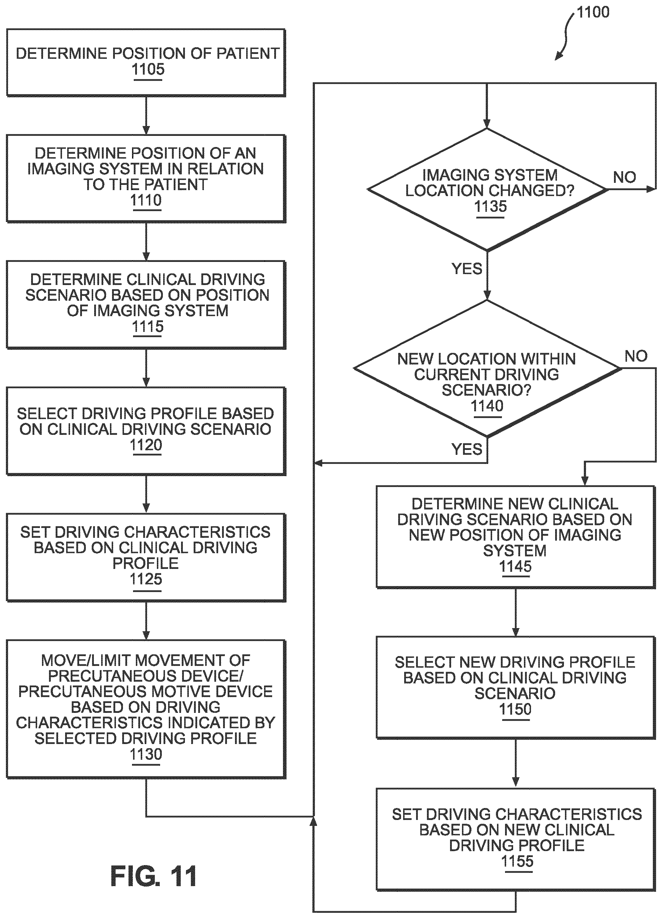

Referring next to FIG. 11, a method 1100 will be discussed in accordance with various example embodiments. As illustrated by block 1105, a position of a patient is determined. As illustrated by block 1110, a position of an imaging system in relation to the patient is determined. In some example embodiments, determining the position of the imaging system includes, but is not limited to, determining a position of an arm of the imaging system.

As illustrated by block 1115, a clinical driving scenario is determined based on the position of the imaging system, *e.g.* the arm of the imaging system. As illustrated by block 1120, a driving profile is selected based on the driving scenario. As illustrated by block 1125, the driving characteristics of a percutaneous device are set based on the clinical driving profile. As illustrated by block 1130, the percutaneous device is moved, or movements of the percutaneous device are limited, based on the driving characteristics.

As illustrated by block 1135, the position of the imaging system is monitored to determine if the imaging system moves. If the imaging system does move, as illustrated by block 1140, a check is made to determine if the imaging system's new position requires a new driving scenario, or if the new position still indicates use of the current clinical driving scenario.

As illustrated by block 1145, if the new position of the imaging system is not within the current clinical driving scenario, a new clinical driving scenario is selected, and at block 1150 a new driving profile is selected based on the new clinical driving scenario. As illustrated by block 1155 new driving characteristics for the percutaneous device are set based on the new driving profile.

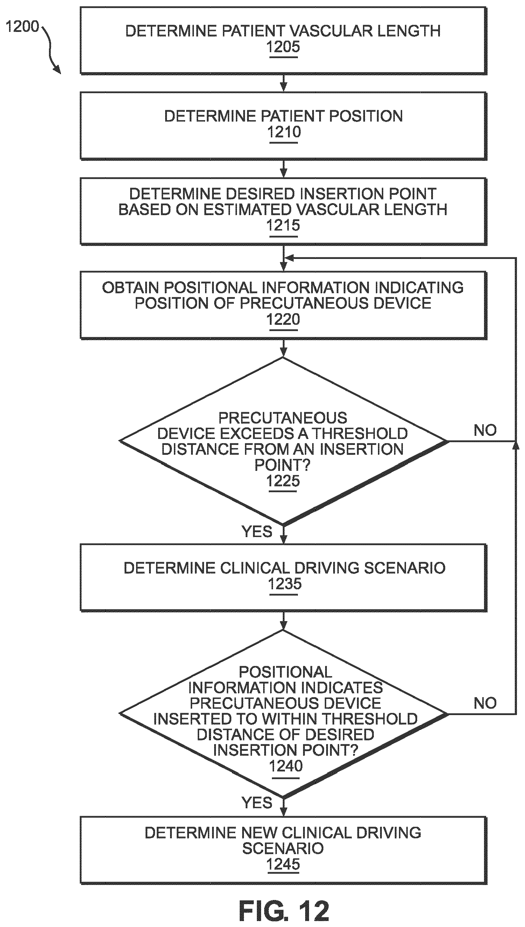

Referring next to FIG. 12 a method 1200 will be discussed in accordance with various example embodiments. As illustrated by block 1205, a patients vascular length is determined. In some example embodiments, the vascular length may be estimated based on a patient's physical characteristics such as height. In other example embodiments, imaging may be used to determine the patient's vascular length.

As illustrated by block 1210, the patient's position is determined. In various example embodiments, the patient's position may, but need not, be determined based on the patient's placement on a table.

As illustrated by block 1215, a desired insertion point of the percutaneous device is determined based on the patient's vascular length.

As illustrated by block 1220, positional information indicates the position of a percutaneous device. This positional information may be obtained, for example, from sensors included in percutaneous device manipulation system 300.

As illustrated by block 1225, the controller determines whether the percutaneous device exceeds a threshold distance of an insertion point. If the percutaneous device has not exceeded a threshold distance from an insertion point, method 1200 returns to block 1220, and monitors the additional positional information.

As illustrated by block 1235, if the percutaneous device has traveled farther than a threshold distance from the insertion point, a clinical driving scenario is selected.

As illustrated by block 1240, a determination is made regarding whether the positional information indicates that the percutaneous device has been inserted into the patient to a within a threshold distance of the desired insertion point. In at least one example embodiment, the determination is made, at least in part, based on the distance the percutaneous device has traveled and the patient's vascular length. In various example embodiments, verification can be performed using the imaging system.

As illustrated by block 1245, if the percutaneous device has been inserted into the patient to a within a threshold distance of the desired insertion point, a new clinical driving scenario is selected.

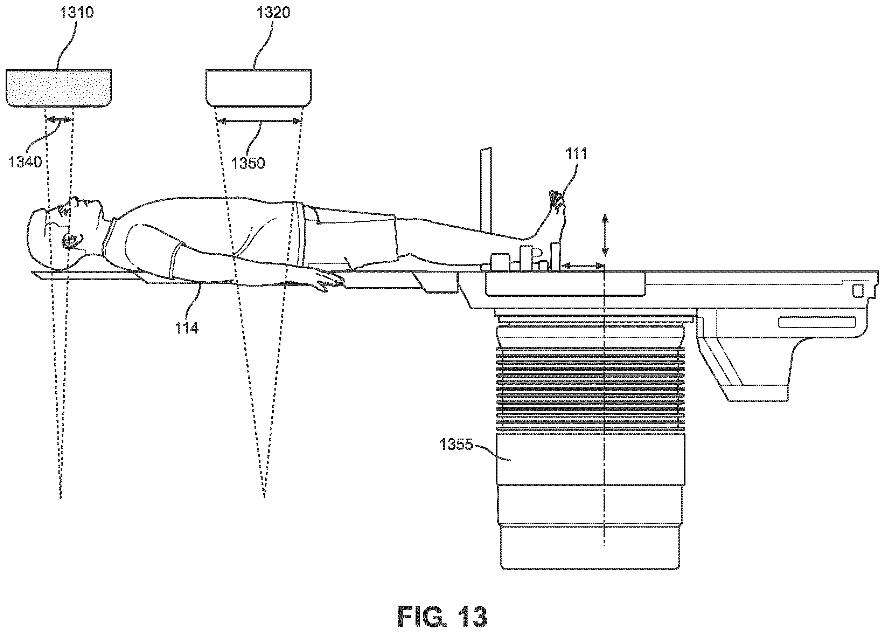

Referring next to FIG. 13, an imaging system field of view (FoV) location in relation to table position will be discussed in accordance with various example embodiments. In the illustrated example embodiment, table 114 may move vertically and/or horizontally relative to table base 1355 to allow positioning of patient 111.

In some example embodiments, an imaging system may include a C-arm used to position the imaging system over particular parts of the patient, and the location of the C-arm may determine the size of the FoV of the imaging system. For example, in first position 1310, the FoV 1340 when the imaging system is positioned near patient's head may be narrower than the FoV 1350 when the imaging system is in a second position 1320 over the patient's torso. In other example embodiments, a physician may be able to decrease the FoV by instructing the imaging system to zoom in. In at least some example embodiments, when the FoV is narrower, a clinical driving scenario can be selected to allow slower/ fine-tuned control. Determination of the FoV in some such embodiments can be achieved through C-Arm Pose information, imaging software parameters, and/or table kinematics data.

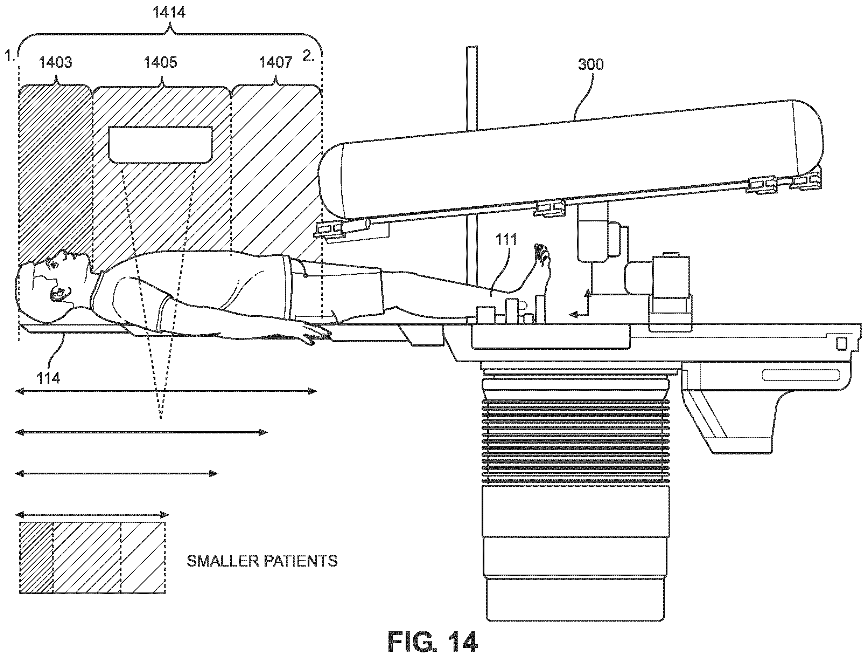

Referring next to FIG. 14 an imaging system field of view (FoV) location in relation to a percutaneous device manipulation system will be discussed in accordance with various example embodiments. In various example embodiments, an imaging system FoV may be based on a location of the imaging device in relation to table position and/or the location of a percutaneous device manipulation system 300 at an introducer site. In some such example embodiments scaled areas with different FoVs may be based on location of the imaging system relative to the table position and/or the location of the percutaneous device manipulation system 300.

In the illustrated example embodiment, area 1414, which runs from the head of the table to the end of percutaneous device manipulation system 300, may be split into three, unequally sized areas 1403, 1405, and 1407 having different FoVs.

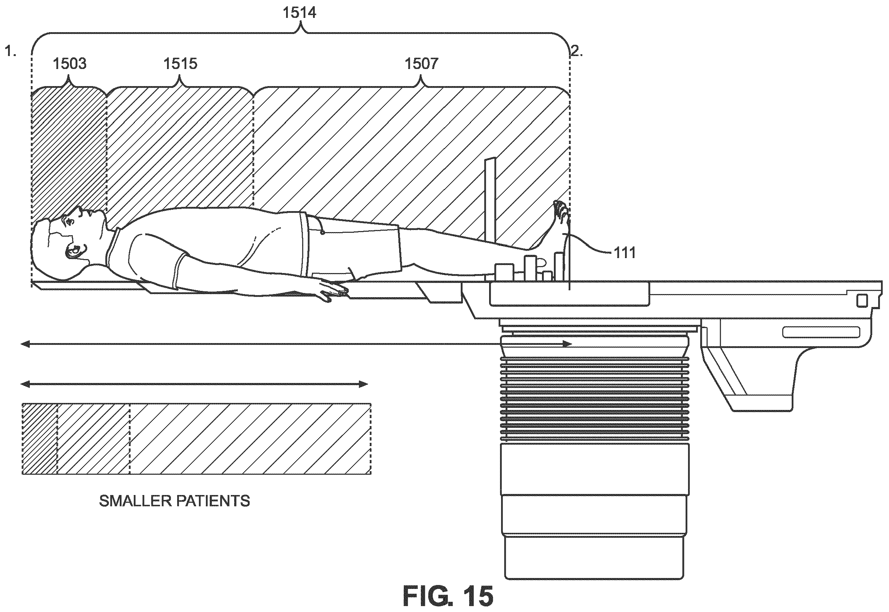

Referring next to FIG. 15 an imaging system field of view (FoV) location in relation patient height and/or table position will be discussed in accordance with various example embodiments. In various example embodiments, an imaging system FoV may be based on a location of the imaging device in relation to table position and/or patient height.

In the illustrated example embodiment, area 1514, which runs from the head of the patient to the patient's fee may be split into three, unequally sized areas 1503, 1505, and 1507 having different FoVs.

In other example embodiments, the distance a percutaneous device has been inserted, and hence adjustment of FoVs and/or selection of clinical drive scenarios, may be determined by patient markers and/or 3D scan data using image based sensors, using fluoroscopic image recognition to determine anatomical markers and device tip position, using opaque markers, or various combinations thereof.

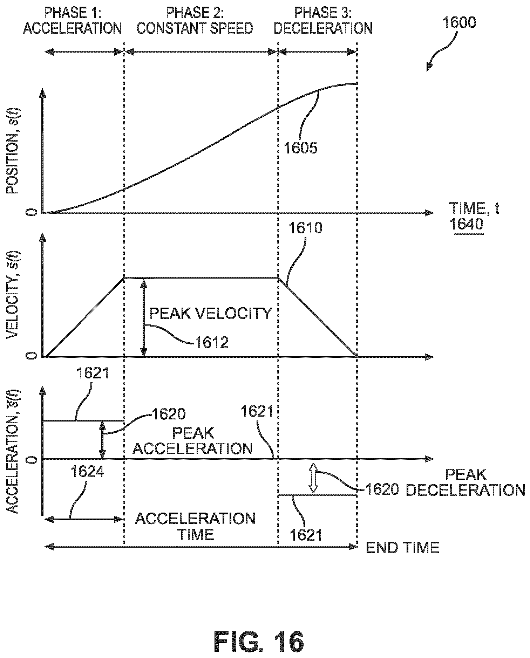

Referring next to FIG. 16 graphs 1600 of percutaneous device driving characteristics will be discussed in accordance with various example embodiments. Graphs 1600 illustrate various driving characteristics associated with particular driving scenarios/driving profiles. In each graph, time moves from left to right. The top graph shows a distance plot 1605, representing a distance a percutaneous device has travelled.

The middle graph shows a velocity plot 1610. Considering distance plot 1605 and velocity plot 1610, it will be noted that the percutaneous device continued moving forward during phase 1, phase 2, and phase 3. It will also be noted that while the device was moving forward, the velocity increased during phase 1 until a peak velocity 1612 was reached during phase 2, and the velocity of the device decreased during phase 3.

Adding the lower graph, which shows what appears to be a discontinuous acceleration plot 1621, it will be noted that during the entirety of phase 1 the percutaneous device was at peak acceleration, during the entirety of phase 3 the device was at peak deceleration, and there no acceleration or deceleration during phase 2. Plot 1621 exhibits a high "jerkiness".

Maximums of any or all of these characteristics may be set by a driving profile associated with a driving scenario, and appropriately controlled by a controller modifying user-control/driving signals. For example, jerkiness may be smoothed using a damping or filtering function and peak velocity may be clipped. In some example embodiments, maximum acceleration may be limited by progressively damping user signals increasing velocity.

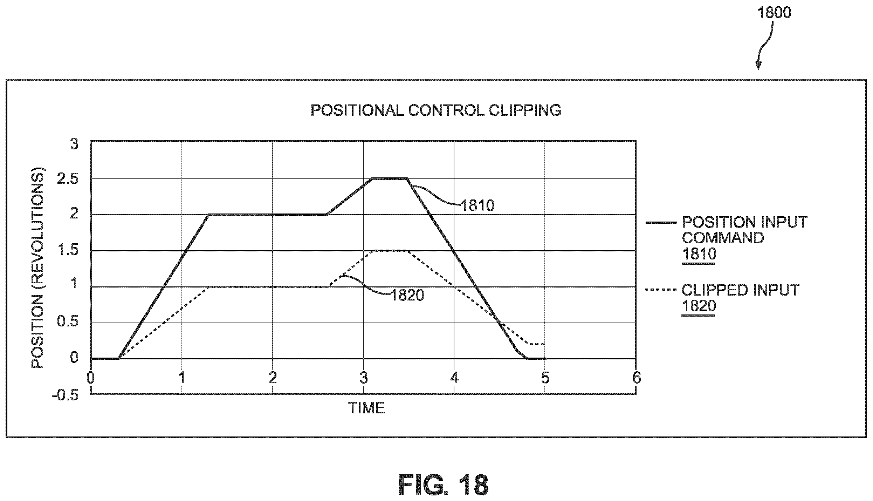

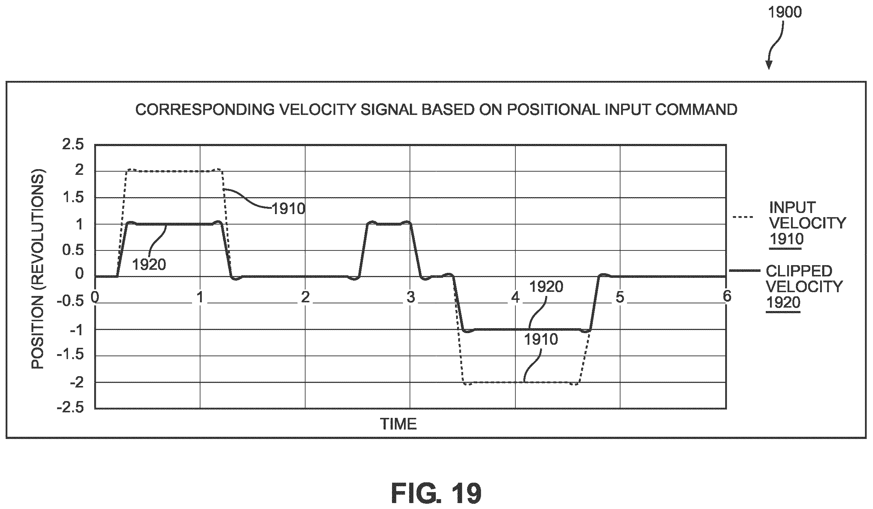

FIGS. 18 and 19, show examples of how changing peak velocity during positional control can result in clipping of a signal. In various example embodiments, limiting peak velocity results in finer control because shorter displacements occur in the same amount of command time. In some example embodiments, a velocity of input positional command signal is clipped/limited to, 1 revolution per second.

In some example embodiments, a percutaneous device position control may be implemented as a rotary dial or other scrolling device capable of measuring driver control input based on a scrolling displacement such as a number of resolutions, an angle of rotation, or the like. A physician may control displacement of a percutaneous device by manipulating the percutaneous device position. In some example embodiments, a displacement per input ratio of 1 scroll=mm. may be used. In at least one example embodiment, an absolute displacement allowed may be set to, for example, 3 rotations.

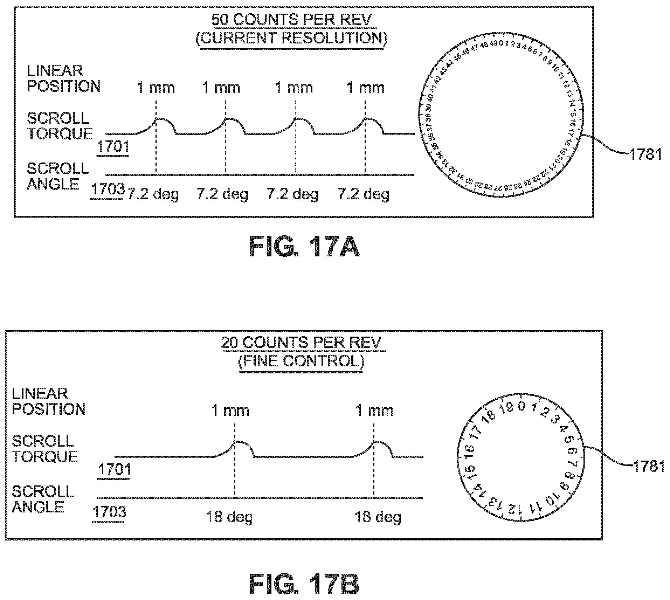

FIGS. 17A and 17B illustrate adjusting a control-resolution of percutaneous device movements using detents. In some such example embodiments, the resolution or tick angle per scroll of a scrolling device 1781 may be changed based on a position of the percutaneous device. For example: for finer control, detents of scrolling device 1781 may be set to 1mm of percutaneous device movement for each of 20 counts per revolution, versus 1mm of percutaneous device movement for each of 50 counts per revolution for coarser control. The increased scroll angle allows a finer degree to mm-of-movement ratio, resulting in more precise control. The scroll torque represents the "detent," or feeling of resistance, when rotating the scroll wheel.

As illustrated by Fig. 17A, with detents of scrolling device 1781 set for 50 counts per revolution (course control), a percutaneous device moves 1mm linearly in response to rotation of scrolling device 1781 by scroll angle 1703, which is 7.2 degrees in FIG. 17A. Note that the numbers illustrated on scrolling device 1781 may not be marked on scrolling device 1781, but are illustrated to show the number of counts per revolution. Scroll torque 1701 may begin to increase just before each detent, which corresponds to 7.2 degrees, and reach its maximum value at each detent. In some example embodiments, the maximum scroll torque 1701 value at the peak of detent may be in the range of 10-20 mNm, although that value may be dependent on a diameter of the scroll wheel or other scrolling mechanism. In some example embodiments, as the scroll angle 1703 continues to increase beyond the detent, which is 7.2 degrees in the illustrated example embodiment, the scroll torque 1701 begins to decrease, and reaches a base level, which may be less than 1 mNm resistance to allow scroll wheel to rotate with minimal resistance, until the next detent is approached - at which point the scroll torque begins to increase again. By contrast, as illustrated by Fig. 17B, detents may be set at a frequency of 20 counts per revolution for fine control, in which detents are set so that scroll torque 1701 increases at each scroll angle 1703 of 18 degrees.

Changing the detents may be performed based on control signals from a controller, such as controller 2040 (Fig. 20) in accordance with a driving profile, and/or based on a position of the percutaneous device. In at least one example embodiment, a haptic knob or other device known to those of ordinary skill in the art may be used to adjust the scroll torque 1701 in response to control signals.

Referring to FIG. 18, a graph 1800 illustrating positional control clipping will be discussed in accordance with various example embodiments. Graph 1800 includes a position input command plot 1810, which represents a signal from a driving input generated in response to a physician's interaction with a driving interface. Clipped input 1820 represents a modified control signal generated by a controller and transmitted to a motive device, for example advance/retract actuator 2072 or 2076 (FIG. 20).

Referring to FIG. 19, a graph 1900 illustrating clipped velocity control signals will be discussed in accordance with various example embodiments. Graph 1900 includes input velocity plot 1910, which represents a signal from a driving input generated in response to a physician's interaction with a driving interface. Clipped input 1920 represents a modified control signal generated by a controller and transmitted to a motive device, for example advance/retract actuator 2072 or 2076 (FIG. 20).

In some example embodiments, device specific characteristics may be included in driving profiles/driving scenarios. For example, driving profiles/driving scenarios may include rotation lockout for devices that specify not to rotate, for example stent retrievers, balloon guides, etc. In some example embodiments, force limits can be included as a driving characteristic. Force limits may be used to change the maximum force a module would apply when a certain device is selected and installed in module. Additionally, driving characteristics may include linear displacement limits for devices that require relative motion between two components, *e.g.* carotid stents.

Referring next to FIG. 20, a percutaneous device control system 2000 will be discussed in accordance with various example embodiments. Various embodiments of percutaneous device control system are described in detail in P.C.T. International Application No. PCT/US2021/070042, filed November 14, 2021, PCT/US2020/041964, filed July 7, 2020, P.C.T. International Application No. PCT/US2009/042720, filed May 4, 2009, PCT/US2020/041923, filed July 14, 2020, U.S. Patent No. 8,480,618, issued July 9, 2013, the entire contents of each of which are incorporated herein by reference. Percutaneous device control system 2000 may include bedside system 112 including various actuating mechanisms that move an associated percutaneous device in response to a user's manipulation of controls 2081. In at least one example embodiment, a control may include a rotary input, such as haptic rotary knob 1781. In the example embodiment shown, bedside system 112 includes a guide wire actuating mechanism 2050, a working catheter actuating mechanism 2052, and a guide catheter actuating mechanism 2054. In other example embodiments, bedside system 112 may include an actuating mechanism for inflating an angioplasty or stent delivery balloon and an actuating mechanism for delivering contrast agent. In the example embodiment shown, guide wire actuating mechanism 2050 and working catheter actuating mechanism 2052 are incorporated within cassette 2056.

Guide wire actuating mechanism 2050 is coupled to guide wire 2058, such that guide wire actuating mechanism 2050 is able to cause guide wire 2058 to advance, retract, and rotate. In various example embodiments, working catheter actuating mechanism 2052 is coupled to working catheter 2060 such that working catheter actuating mechanism 2052 is able to cause working catheter 2060 to advance, retract, and rotate. Guide catheter 2064 is coupled to guide catheter actuating mechanism 2054 such that guide catheter actuating mechanism 2054 is able to cause guide catheter 2064 to advance, retract, and rotate. In various example embodiments, guide wire actuating mechanism 2050, working catheter actuating mechanism 2052, and guide catheter actuating mechanism 2054 may each include an engagement structure suitable for engaging the respective percutaneous device, such that the actuating mechanism is able to impart axial and/or rotational movement to the percutaneous device.

A Y-connector 2066 may be coupled to guide catheter actuating mechanism 2054 via connector 2068. In the example embodiment shown, Y-connector 2066 is connected to cassette 2056. In some example embodiments, Y-connector 2066 includes a first leg, a second leg, and a third leg. The first leg of the Y-connector 2066 may be connected to, or in communication with, an internal lumen of guide catheter 2064. The second leg may be angled away from the longitudinal axis of guide catheter 2064 and provide a port for the injection of fluids *(e.g.,* contrast media, medicine, etc.) into the lumen of guide catheter 2064. The third leg of Y-connector 2066 is coupled to a cassette 2056 and receives both guide wire 2058 and working catheter 2060. By this arrangement, guide wire 2058 and working catheter 2060 are inserted through Y-connector 2066 into the internal lumen of guide catheter 2064. A hemostasis valve and Y-connector that may be used in the present invention are shown in US 9,452,277 and are incorporated herein by reference in its entirety.

In the illustrated example embodiment, guide wire actuating mechanism 2050 includes a rotate actuator 2070 and an advance/retract actuator 2072. Rotate actuator 2070 may be configured to cause rotation of guide wire 2058 about its longitudinal axis. Advance/retract actuator 2072 may be configured to advance and/or retract guide wire 2058 (i.e., to advance and/or retract along the longitudinal axis of the guide wire) within patient 111. Working catheter actuating mechanism 2052 includes a rotate actuator 2074 and an advance/retract actuator 2076. Rotate actuator 2074 is configured to cause rotation of working catheter 2060 about its longitudinal axis. Advance/retract actuator 2076 is configured to advance and/or retract working catheter 2060 (*e.g.,* to advance and/or retract along the longitudinal axis of the working catheter) within patient 111.

In various example embodiments, guide catheter actuating mechanism 2054 includes a rotate actuator 2078, an advance/retract actuator 2080, and a bend actuator 2082. Rotate actuator 2078 is configured to cause rotation of guide catheter 2064 about its longitudinal axis. Advance/retract actuator 2080 is configured to advance and/or retract guide catheter 2064 *(e.g.,* to advance and/or retract along the longitudinal axis of the guide catheter) within patient 111.

In some embodiments, guide catheter 2064 may include one or more bend control elements that allow the user to cause bending of the distal tip of guide catheter 2064. In such an embodiment, bend actuator 2082 may cause the distal tip of guide catheter 2064 to bend in response to a user's manipulation of controls 2081. An example of one bend control element that may be used with the present invention is shown in US 2024/0024055, which is incorporated by reference in its entirety.

In various example embodiments, controls 2081 and controller 2040 are located at workstation 2014 are communicably coupled to various portions of bedside system 112 to allow the user to control movement of guide wire 2058, working catheter 2060 and guide catheter 2064 and any other percutaneous devices included in bedside system 112. In some example embodiments, optional controller 1240a may be included in bedside system 112, or at another location outside of workstation 2014, in addition to or in place of controller 2040 at workstation 2014. In at least one example embodiment functionality of optional controller 1240a is the same as that described for controller 2040.

In the illustrated example embodiment, controls 2081 and controller 2040 are coupled to guide catheter actuating mechanism 2054 to allow the user to move guide catheter 2064. In addition, controls 2081 are coupled to cassette 2056 to allow the user to control guide wire 2058 via guide wire actuating mechanism 2050 and to control working catheter 2060 via working catheter actuating mechanism 2052.

In some example embodiments, cassette 2056 is configured to be coupled to a motor drive base. In this embodiment, each of the actuators 2070, 2072, 2074, and 2076 of cassette 2056 are configured to engage capstans extending from the motor drive base. Motors located within the motor drive base drive (e.g., rotate) the capstans which in turn drive the actuators 2070, 2072, 2074, and 2076 of cassette 2056. When the actuators 2070, 2072, 2074, and 2076 of cassette 2056 are engaged with guide wire 2058 and working catheter 2060, respectively, the actuators 2070, 2072, 2074, and 2076 of cassette 2056 transfer the rotational movement of the capstans to cause the movement of guide wire 2058 and working catheter 2060. In another embodiment, the motors that drive the capstans of the motor drive base may be located outside of the base connected to cassette 2056 via an appropriate transmission device *(e.g.,* shaft, cable, etc.). In yet another embodiment, cassette 2056 includes motors located within cassette 2056 associated with the actuators 2070, 2072, 2074, and 2076, and cassette 2056 is mounted to a base providing a power supply *(e.g.,* battery, AC building power supply, etc.) to the motors within cassette 2056.

Referring to FIG. 21, a processing system 2040 will be discussed in accordance with various example embodiments. Processing system 2040 may be an electronic control unit configured to provide percutaneous device control system 2000 with the various functionalities described herein. For example, processing system 2040 may be an embedded system, a dedicated circuit, a general-purpose system programmed with the functionality described herein, etc. Processing system 2040 includes a processing circuit 2190, memory 2192, communication module or subsystem 2194, communication interface 2196, procedure control module or subsystem 2198, simulation module or subsystem 2103, assist control module or subsystem 2102, mode selection module or subsystem 2104, inventory module or subsystem 2106, GUI module or subsystem 2108, data storage module or subsystem 110, and record module or subsystem 2112.

Processing circuit 2190 may be a general-purpose processor, an application specific processor (ASIC), a circuit containing one or more processing components, a group of distributed processing components, a group of distributed computers configured for processing, etc., configured provide the functionality of module or subsystem components. Memory 2192 (*e.g.,* memory unit, memory device, storage device, etc.) may be one or more devices for storing data and/or computer code for completing and/or facilitating the various processes described in the present disclosure.

Memory 2192 may include volatile memory and/or nonvolatile memory. Memory 2192 may include database components, object code components, script components, and/or any other type of information structure for supporting the various activities described example embodiments. According to an example embodiment, any distributed and/or local memory device of the past, present, or future may be utilized with the systems and methods of this disclosure. According to an example embodiment, memory 2192 is communicably connected to processing circuit 2190 and module components, *e.g.,* via a circuit or any other wired, wireless, or network connection) and includes computer code for executing one or more processes described herein. A single memory unit may include a variety of individual memory devices, chips, disks, and/or other storage structures or systems.

Any or all of the module or subsystem components may include computer code (*e.g.,* object code, program code, compiled code, script code, executable code, or any combination thereof), hardware, software, or any combination thereof, for conducting each module's respective functions. One or more portions of any or all of the module components may be stored in memory 2192, or in one or more local, distributed, and/or remote memory units configured to be in communication with processing circuit 2190 or another suitable processing system.

In various example embodiments, communication interface 2196 includes one or more component for communicably coupling processing system 2040 to the other components of percutaneous device control system 2000 via communication links. Communication interface 2196 may include one or more jacks or other hardware for physically coupling communication links to processing system 2040, an analog to digital converter, a digital to analog converter, signal processing circuitry, and/or other suitable components. Communication interface 2196 may include hardware configured to connect processing system 2040 with the other components of percutaneous device control system 2000 via wired or wireless connections. Communication module 2194 is configured to support the communication activities of processing system 2040 (*e.g.,* negotiating connections, communication via standard or proprietary protocols, etc.).

Data storage module 110 is configured to support the storage and retrieval of information by processing system 2040. In one or more example embodiments data storage module 2110 is a database for storing patient specific data, including image data. In another embodiment, data storage module 2110 may be located on a hospital network. Data storage module 2110 and/or communication module 2194 may also be configured to import and/or export patient specific data from hospital network for use by processing system 2040.

In various example embodiments, processing system 2040 also includes a procedure control module 2198 configured to support the control of bedside system 112 during a catheter based medical procedure. Procedure control module 2198 allows the user to operate bedside system 112 by manipulating controls 2081. In various embodiments, procedure control module 2198 is configured to generate one or more control signals 2016 based upon the user's manipulation of controls 2081 and/or other data available to procedure control module 2198. In some example embodiments, the control module 2198 may alter, inhibit, clip, or otherwise modify signals received from controls 2081 to generate control signals 2016.

As shown in FIG. 20, control signals 2016, generated by procedure control module 2198, are communicated from processing system 2040 to the various actuators of bedside system 112. In response to control signals 2016, the actuators of cassette 2056 cause movement of the guide wire, working catheter and/or guide catheter. Procedure control module 2198 may also cause data appropriate for a particular procedure to be displayed on monitors. Procedure control module 2198 may also cause various icons to be displayed on a touch screen that the user may interact with to control the use of bedside system 112. In some example embodiments, procedure control module 98 is configured to cause bedside system 112 to move (*e.g.,* set or adjust a rate of movement or rotation in response to a particular input received by controls 2081, as modified by a driving profile associated with a clinical driving scenario. In one or more example embodiments, procedure control module 98 may be configured such that when guide wire control 23 is actuated, bedside system 112 causes the guide wire to advance, retract or rotate at a rate determined, in part, by a user's interaction with controls 2081, and in part by driving characteristics associated with a current clinical driving scenario.

In some example embodiments, the movement rate of a percutaneous device caused by bedside system 112 is proportional to the amount of displacement of the control as modified by information included in a driving profile associating clinical driving scenarios with particular driving characteristics. For example, where controls 23, 25 and 29 are joystick controls, the movement rate of a percutaneous device caused by bedside system 112 may be a function of the degree of displacement of the joystick from the resting position.

In the illustrated example embodiment, processing system 2040 includes a GUI module 2108 that controls the display of various information on one or more display devices. In various example embodiments, GUI module 2108 may be configured to display image data captured by imaging system 12 included in catheter procedure system 110. In one example embodiment, shown in FIG. 2, GUI module 2108 may be configured to cause a monitor or other display device, to display an image of a portion of a patient 111 during a catheterization procedure.

### Non-Limiting Illustrative Embodiments

In illustrative embodiment 1, a controller comprises: at least one processor; memory coupled to the at least one processor, the memory storing computer-executable instructions; and wherein the at least one processor is configured to execute the computer-executable instructions to cause the controller to determine a clinical driving scenario associated with a percutaneous device, and set driving characteristics associated with the percutaneous device based on the clinical driving scenario and information included in a driving profile, wherein the information included in the driving profile includes information associating particular clinical driving scenarios with particular driving characteristics.

Illustrative embodiment 2 includes the controller of embodiment 1, wherein the at least one processor is further configured to execute the computer-executable instructions to cause the controller to: determine the clinical driving scenario based on positional information indicating a distance the percutaneous device is inserted in a patient, a comparison of the distance the percutaneous device is inserted into the patient and an estimated patient vascular length wherein the positional information includes at least one of, information indicating a position of an arm of an imaging system relative to a patient, a location of a percutaneous device manipulation system relative to the patient, a position of a cartridge along a drive-axis of the percutaneous device manipulation system, image recognition of a marker on the patient, or image recognition of a marker on the percutaneous device.

Illustrative embodiment 3 includes the controller of embodiment 1 or 2, wherein the at least one processor is further configured to execute the computer-executable instructions to cause the controller to: receive user control signals from a driver interface, wherein the user control signals indicate requested movement of a percutaneous device being controlled by the controller; and set the driving characteristics of the percutaneous device by modifying the user control signals based on the clinical driving scenario and the information included in the driving profile.

Illustrative embodiment 4 includes the controller of any of embodiments 1-3, wherein the at least one processor is further configured to execute the computer-executable instructions to cause the controller to: determine the clinical driving scenario based on at least one of a field of view of an imaging system or a position of an imaging system relative to a patient, wherein the field of view of the imaging system represents a zone between an access site at which the percutaneous device is inserted into the patient and the head of the patient.

Illustrative embodiment 5 includes the controller of any of embodiments 1-4, wherein the information included in the driving profile includes: information adjusting the particular driving characteristics under the particular clinical driving scenarios.

Illustrative embodiment 6 includes the controller of any of embodiments 1-5, wherein the particular clinical driving scenarios include: at least one of a type of percutaneous device, a configuration of the percutaneous device, a distance the percutaneous device is inserted in a patient, or whether a distal portion of the percutaneous device is still fully within an existing percutaneous device.

Illustrative embodiment 7 includes the controller of any of embodiments 1-6, wherein the particular driving characteristics include: at least one of a peak velocity, a peak acceleration, jerkiness, a peak rotational velocity, a peak rotational acceleration, a displacement per input ratio, a maximum absolute displacement, a maximum force, a maximum linear displacement, or a rotational lockout.

Illustrative embodiment 8 includes the controller of any of embodiments 1-7, wherein the at least one processor is further configured to execute the computer-executable instructions to cause the controller to: determine a distance a percutaneous device is inserted in a patient, receive user control signals from a driver interface, wherein the driver interface is configured to respond to user interaction with the driver interface to control movement of the percutaneous device, generate adjusted user control signals based on the distance the percutaneous device is inserted in the patient, and transmit the adjusted user control signals to a motive device configured to move the percutaneous device in accordance with the adjusted user control signals.

Illustrative embodiment 9 includes the controller of any of embodiment 8, wherein the at least one processor is further configured to execute the computer-executable instructions to cause the controller to: determine the distance the percutaneous device is inserted in the patient based on one or more of kinematic information, a position of an imaging system relative to a patient, a location of a percutaneous device manipulation system relative to the patient, or a displacement of a cartridge along a drive-axis of a percutaneous device manipulation system.

Illustrative embodiment 10 includes the controller of any of embodiments 8-9 wherein the at least one processor is further configured to execute the computer-executable instructions to cause the controller to: generate the adjusted user control signals by altering the user control signals based on at least one of a field of view of an imaging system, or a position of an imaging system relative to a patient, wherein the field of view of the imaging system represents a zone between an access site at which the percutaneous device is inserted into the patient and the head of the patient.

Illustrative embodiment 11 includes the controller of any of embodiments 8-10, wherein the at least one processor is further configured to execute the computer-executable instructions to cause the controller to: generate the adjusted user control signals based on profile information included in a driving profile, wherein the profile information included in the driving profile includes first information associating the distance the percutaneous device is inserted in the patient with movement characteristics of the percutaneous device.

Illustrative embodiment 12 includes the controller of any of embodiments 8-11, wherein the profile information included in the driving profile further includes: second information associating the characteristics of the percutaneous device with at least one of a percutaneous device safe-loading position, a percutaneous device type, or a configuration of the percutaneous device.

Illustrative embodiment 13 includes the controller of any of embodiments 8-12, wherein: movement characteristics of the percutaneous device include at least one of a peak velocity limit, a peak acceleration limit, a jerkiness limit, a rotational velocity limit, a displacement per input ratio, an absolute displacement limit, a rotational lockout, a maximum force limit, or a linear displacement limit.

Illustrative embodiment 14 includes a robotic device comprising: a motor configured to impart motion to a percutaneous device in response to motor control signals; a driver interface configured to transmit user control signals to a controller in response to user manipulation of the driver interface; and the controller of any of embodiments 1-13.

Illustrative embodiment 15 includes a method comprising: determining a clinical driving scenario associated with a percutaneous device, and setting driving characteristics associated with the percutaneous device based on the clinical driving scenario and information included in a driving profile, wherein the information included in the driving profile includes information associating particular clinical driving scenarios with particular driving characteristics.

As discussed herein, the terminology "one or more" and "at least one" may be used interchangeably. Furthermore, although the terms first, second, etc. may be used herein to describe various elements, these elements should not be limited by these terms. These terms are only used to distinguish one element from another. For example, a first element could be termed a second element, and similarly, a second element could be termed a first element, without departing from the scope of this disclosure. As used herein, the term "and/or," includes any and all combinations of one or more of the associated listed items.

When an element is referred to as being "connected," or "coupled," to another element, it can be directly connected or coupled to the other element or intervening elements may be present. By contrast, when an element is referred to as being "directly connected," or "directly coupled," to another element, there are no intervening elements present. Other words used to describe the relationship between elements should be interpreted in a like fashion *(e.g.,* "between," versus "directly between," "adjacent," versus "directly adjacent," etc.).

The terminology used herein is for the purpose of describing particular embodiments only and is not intended to be limiting. As used herein, the singular forms "a," "an," and "the," are intended to include the plural forms as well, unless the context clearly indicates otherwise. It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof.

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

Specific details are provided in the preceding description to provide a thorough understanding of example embodiments. However, it will be understood by one of ordinary skill in the art that example embodiments may be practiced without these specific details. For example, systems may be shown in block diagrams so as not to obscure the example embodiments in unnecessary detail. In other instances, well-known processes, structures, and techniques may be shown without unnecessary detail in order to avoid obscuring example embodiments.

As discussed herein, illustrative embodiments have been described with reference to acts and symbolic representations of operations *(e.g.,* in the form of flow charts, flow diagrams, data flow diagrams, structure diagrams, block diagrams, etc.) that may be implemented as program modules or functional processes include routines, programs, objects, components, data structures, etc., that perform particular tasks or implement particular abstract data types and may be implemented using existing hardware at, for example, existing user equipment or other network elements and/or hardware. Such existing hardware may be processing or control circuitry such as, but not limited to, one or more processors, one or more Central Processing Units (CPUs), one or more controllers, one or more arithmetic logic units (ALUs), one or more digital signal processors (DSPs), one or more microcomputers, one or more field programmable gate arrays (FPGAs), one or more System-on-Chips (SoCs), one or more programmable logic units (PLUs), one or more microprocessors, one or more Application Specific Integrated Circuits (ASICs), or any other device or devices capable of responding to and executing instructions in a defined manner.

Although a flow chart may describe the operations as a sequential process, many of the operations may be performed in parallel, concurrently, or simultaneously. In addition, the order of the operations may be re-arranged. A process may be terminated when its operations are completed, but may also have additional steps not included in the figure. A process may correspond to a method, function, procedure, subroutine, subprogram, etc. When a process corresponds to a function, its termination may correspond to a return of the function to the calling function or the main function.

As disclosed herein, the term "storage medium," "computer readable storage medium" or "non-transitory computer readable storage medium" may represent one or more devices for storing data, including read only memory (ROM), random access memory (RAM), magnetic RAM, core memory, magnetic disk storage mediums, optical storage mediums, flash memory devices and/or other tangible machine-readable mediums for storing information. The term "computer-readable medium" may include, but is not limited to, portable or fixed storage devices, optical storage devices, and various other mediums capable of storing, containing, or carrying instruction(s) and/or data.

Furthermore, example embodiments may be implemented by hardware, software, firmware, middleware, microcode, hardware description languages, or any combination thereof. When implemented in software, firmware, middleware or microcode, the program code or code segments to perform the necessary tasks may be stored in a machine or computer readable medium such as a computer readable storage medium. When implemented in software, a processor or processors will perform the necessary tasks. For example, as mentioned above, according to one or more example embodiments, at least one memory may include or store computer program code, and the at least one memory and the computer program code may be configured to, with at least one processor, cause a network element or network device to perform the necessary tasks. Additionally, the processor, memory, and example algorithms, encoded as computer program code, serve as means for providing or causing performance of operations discussed herein.

A code segment of computer program code may represent a procedure, function, subprogram, program, routine, subroutine, module, software package, class, or any combination of instructions, data structures or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable technique including memory sharing, message passing, token passing, network transmission, etc.

The terms "including" and/or "having," as used herein, are defined as comprising (*i.e.,* open language). The term "coupled," as used herein, is defined as connected, although not necessarily directly, and not necessarily mechanically. Terminology derived from the word "indicating" (*e.g.,* "indicates" and "indication") is intended to encompass all the various techniques available for communicating or referencing the object/information being indicated. Some, but not all, examples of techniques available for communicating or referencing the object/information being indicated include the conveyance of the object/information being indicated, the conveyance of an identifier of the object/information being indicated, the conveyance of information used to generate the object/information being indicated, the conveyance of some part or portion of the object/information being indicated, the conveyance of some derivation of the object/information being indicated, and the conveyance of some symbol representing the object/information being indicated.

According to example embodiments, user equipment, other network elements, or the like, may be (or include) hardware, firmware, hardware executing software or any combination thereof. Such hardware may include processing or control circuitry such as, but not limited to, one or more processors, one or more CPUs, one or more controllers, one or more ALUs, one or more DSPs, one or more microcomputers, one or more FPGAs, one or more SoCs, one or more PLUs, one or more microprocessors, one or more ASICs, or any other device or devices capable of responding to and executing instructions in a defined manner.

Benefits, other advantages, and solutions to problems have been described above with regard to specific embodiments of the invention. However, the benefits, advantages, solutions to problems, and any element(s) that may cause or result in such benefits, advantages, or solutions, or cause such benefits, advantages, or solutions to become more pronounced are not to be construed as a critical, required, or essential feature or element of any or all the claims.

## Claims

1. A controller comprising:
at least one processor;
memory coupled to the at least one processor, the memory storing computer-executable instructions; and
wherein the at least one processor is configured to execute the computer-executable instructions to cause the controller to
determine a clinical driving scenario associated with a percutaneous device, and
set driving characteristics associated with the percutaneous device based on the clinical driving scenario and information included in a driving profile, wherein the information included in the driving profile includes information associating particular clinical driving scenarios with particular driving characteristics.

2. The controller as in claim 1, wherein the at least one processor is further configured to execute the computer-executable instructions to cause the controller to:
determine the clinical driving scenario based on positional information indicating a distance the percutaneous device is inserted in a patient.

3. The controller as in claim 1 or 2, wherein the at least one processor is further configured to execute the computer-executable instructions to cause the controller to:
determine the clinical driving scenario based on a comparison of the distance the percutaneous device is inserted into the patient and an estimated patient vascular length.

4. The controller as in claim 2 or 3, wherein:
the positional information includes at least one of, information indicating a position of an arm of an imaging system relative to a patient, a location of a percutaneous device manipulation system relative to the patient, a position of a cartridge along a drive-axis of the percutaneous device manipulation system, image recognition of a marker on the patient, or image recognition of a marker on the percutaneous device.

5. The controller as in any of claims 1-4, wherein the at least one processor is further configured to execute the computer-executable instructions to cause the controller to:
determine the clinical driving scenario based on at least one of a field of view of an imaging system or a position of an imaging system relative to a patient, wherein the field of view of the imaging system represents a zone located between an access site at which the percutaneous device is inserted into the patient and the head of the patient.

6. The controller as in any of claims 1-5, wherein the particular clinical driving scenarios include:
at least one of a type of percutaneous device, a configuration of the percutaneous device, a distance the percutaneous device is inserted in a patient, or whether a distal portion of the percutaneous device is still fully within an existing percutaneous device.

7. The controller as in any of claims 1-6, wherein the particular driving characteristics include:
at least one of a peak velocity, a peak acceleration, jerkiness, a peak rotational velocity, a peak rotational acceleration, a displacement per input ratio, a maximum absolute displacement, a maximum force, a maximum linear displacement, or a rotational lockout.

8. The controller as in any of claims 1-7, wherein the at least one processor is configured to execute the computer-executable instructions to cause the controller to
determine a distance a percutaneous device is inserted in a patient,
receive user control signals from a driver interface, wherein the driver interface is configured to respond to user interaction with the driver interface to control movement of the percutaneous device,
generate adjusted user control signals based on the distance the percutaneous device is inserted in the patient, and
transmit the adjusted user control signals to a motive device configured to move the percutaneous device in accordance with the adjusted user control signals.

9. The controller as in any of claims 1-8, wherein the at least one processor is further configured to execute the computer-executable instructions to cause the controller to:
determine the distance the percutaneous device is inserted in the patient based on kinematic information.

10. The controller as in any of claims 1-9, wherein the at least one processor is further configured to execute the computer-executable instructions to cause the controller to:
generate the adjusted user control signals based on profile information included in a driving profile, wherein the profile information included in the driving profile includes first information associating the distance the percutaneous device is inserted in the patient with limits to be imposed on movement of the percutaneous device.

11. The controller as in any of claims 1-10, wherein the profile information included in the driving profile further includes:
second information associating the limits to be imposed on movement of the percutaneous device with at least one of a percutaneous device safe-loading position, a percutaneous device type, or a configuration of the percutaneous device.

12. The controller as in any of claims 1-11, wherein:
the limits to be imposed on the movement of the percutaneous device include at least one of a peak velocity limit, a peak acceleration limit, a jerkiness limit, a rotational velocity limit, a displacement per input ratio, an absolute displacement limit, a rotational lockout, a maximum force limit, or a linear displacement limit.

13. A robotic device comprising:
a motor configured to impart motion to a percutaneous device in response to motor control signals;
a driver interface configured to transmit user control signals to a controller in response to user manipulation of the driver interface; and
a controller as in any of claims 1-12.

14. The robotic device as in claim 13, wherein the motor is further configured to:
impart the motion to the percutaneous device by moving a cartridge coupled to the motor along a drive-axis in response to the motor control signals, wherein movement of the cartridge moves the percutaneous device.

15. A method comprising:
determining a clinical driving scenario associated with a percutaneous device; and
setting driving characteristics associated with the percutaneous device based on the clinical driving scenario and information included in a driving profile, wherein the information included in the driving profile includes information associating particular clinical driving scenarios with particular driving characteristics.
